# DEMANDE DE BREVET EUROPEEN

(11) **EP 2 684 588 A1**
(43) Date de publication de la demande: **15.01.2014**
(21) Numéro de dépôt: 13366004.3
(22) Date de dépôt: 11.07.2013
(51) Int. Cl.: B01D 29/50, B01D 33/35, A01N 63/00

(54) **Filtres macro/microporeux pour l'incubation et le diagnostique de l'activité microbiologique d'echantillons environnementaux**

(30) Priorité: 13.07.2012 FR 1201990
(71) Demandeur: Polyor SARL, 54000 Nancy (FR)
(72) Inventeur: Claude, Pierre-Philippe, 54000 Nancy (FR)

(57) **Abrégé**

Filtre macro-microporeux constitué d'un feuilleté multi-plis semi-rigide comprenant quatre couches ;
➢ (a) n'étant pas en contact avec l'échantillon est étanche ;
➢ (b) placée sous la première est microporeuse du fait d'une maille ou d'une aperture ne dépassant pas avantageusement 0,45 microns (um) ;
➢ (c) placée sous la deuxième est macroporeuse du fait d'une maille ou d'une aperture avantageusement d'au moins 70 microns et/ou toute en ne permettant pas le passage de particules de dimensions supérieures à 500 um ;
➢ (d) placée sous la troisième est elle soit étanche, soit microporeuse de manière à assurer la stérilité préétablie des deuxième et troisième couches.

Les couches (a), (b) et (d) sont sous la forme d'opercules rétractibles, la couche (c) devenant à terme orpheline une fois les couches (d), (a) et (b) retirées dans cette ordre. Il est ainsi possible d'apporter - aseptiquement, à l'échantillon de l'eau et/ou une solution nutritive sans avoir à manipuler directement l'échantillon et/ou rompre le lien aseptique qui le protège. Le feuilleté de dimension d1 est placé à la surface d'un bac de dimension d2 inférieure à d1 de manière à le fixer sur les parois dudit bac. Le bac contient un substrat cellulosique enrichit d'oses à hauteur de ∼100 uM et séparé de l'échantillon par une pellicule étanche et hydrophile. Ledit bac est par la suite vissé à un récipient de manière à permettre la suspension aqueuse des particules supérieures à 45 um mais inférieures à 500 microns.

## Description

### DOMAINE TECHNIQUE DE L'INVENTION

Microbiologie et fertilité de sols arables appliquée à l'agronomie. Il est question de protocoles et/ou de kits de diagnostique de sols pour la préconisation d'itinéraire techniques, y compris l'apport d'engrais bactériennes et/ou (organo)minéraux. Le domaine technique concerne aussi la plasturgie dédiée à la fabrication de tels kits.

### ÉTAT DE LA TECHNIQUE

### 1. Diagnostique des populations bactériennes in situ (Tableaux 1 et 2)

Le diagnostique physiologique et génomique des populations bactériennes de sols est recherché en agronomie (Tableau 1), notamment en biofertilisation par (ré)introduction de *bactéries favorisant la croissance des plantes* (BFCP).

**Tableau 1 : Approches et/ou kits pour le diagnostique microbiologique des sols**

| **Approche, Diagnostique, kit, etc.** | **Références** |
|---|---|
| Metabolic quotient | Degens et Harris 1997 ; Sawada et al. 2009 |
| Substrat induced respiration | Degens et Harris 1997 ; Sawada et al. 2009 |
| In situ catabolic potentiel | Degens 1998 |
| Catabolic response profils | Degens 1999 |
| Community level physiological profils | Wakelin et al. 2008 ; Campbell et al. 2003 ; Rowell 1995 |
| Catabolic diversity | Degens et Harris 1997 ; Sawada et al. 2009 |
| Métabolic diversity | Degens et Harris 1997 ; Sawada et al. 2009 |
| Functional divesity | Griffiths et al. 2001 |
| BioLog | Smalla et al. 1998 |
| MicroResp (cf. CLPP) | http://www.microresp.com/ |

Campbell et al. 2003 et Rowell 1995 proposent une approche basée sur le *substrat induced respiration* (SIR ; induction métabolique) selon le type de substrat (sucres, acides aminées, alcools, etc.) suivi d'une *analyse par composantes principales* (ACP). Le problème est que l'apport de quelques 100 à 120 mg-C / g-sol selon cette approche équivaut à plus de 150 tonnes de sucres apportés par hectare. De tels apports de sucres équivalent à des retours de résidus de culture pailleux de l'ordre de 700 tonnes, soit plus de 100 fois celui d'une culture céréalière. Wakelin et al. 2008, à l'aide de la méthode dite MicroResp™ inspirée de l'approche Biolog-CLPP (*community level physiological profile*), rapportent surtout une caractérisation des sols selon la structure des communautés bactériennes les plus *zymogènes* (Langer et al. 2004). Paradoxalement, cette appréciation via l'apports d'importantes quantités de substrats glucidiques et aminés révèle surtout les populations (azoto)bactériennes les moins réactives à de faibles doses de tels substrats, faibles doses caractéristiques de l'environnement oligotrophique qu'est la zone vadose des sols arables. Pire, les quantités de sol, frais ou sec, utilisés pour ce type de caractérisation métabolique (catabolique) sont relativement (trop) petites, soit de l'ordre de quelques grammes (Tableau 2) ;

**Tableau 2 : Quantités de sol préconisés pour la caractérisation métabolique de sols arables**

| **quantité (g)** | **frais (F), sec (S)** | **Référence** |
|---|---|---|
| 0,45 | F | Campbell et al. 2003 / Wakelin et al. 2008 |
| 1 | S | Degens et al. 1997, 1998, 1999 |
| 1,7 | F | Hill et al. 2008 |
| 2 | F | Nguyen et Gurckert 2001 |
| 4 | F | Nguyen et Gurckert 2001 |
| 4 | F | Nguyen et Gurckert 2001 |
| 15 | F | Aldén et al. 2001 |
| 25 | S | De Nobili et al. 2001 |
| 30 | S | Coddy et al. 1986 |
| 30 | S | Sawada et al. 2009 |
| 30 | S | Schneckenberger et al. 2008 |
| 50 | S | Mondini et al. 2006 |
| 50 | S | Cayuela et al. 2009 |
| 0,5 | F | Mettel et al. 2010 |
| 0,4 | F | Chen et al. 2007 |
| 0,5 | F | Bürgmann et al. 2003 |
| 0,5 | F | Bodrossy et al. 2006 |
| 1 g sol/10 ml d'eau | F | Fleming et al. 1998 |
| 10 | F | Tsai et al. 1991 |
| ? (*cf*. Fast Spin) | F | Kolb et al. 2003 |
| 1,5 | F | Mendum et al. 1998 |
| 4 | F | Hurt et al. 2001 |
| 1 | F | Ranjard et al. 1998 |

Pourtant, il existe actuellement bon nombre de « kit » pour l'extraction et/ou le dosage et/ou la purification de l'ADN et/ou de l'ARN extraites directement de cellules et/ou d'échantillons de sols, d'eau, de sédiments marins ou telluriques, etc. (Tableau 3) ;

**Tableau 3 : Approches et/ou kits d'extraction et/ou de dosage de l'ADN et/ou l'ARN de sols.**

| |
|---|
| (Roche™ mRNA-Isolation Kit) |
| (MOBio™) |
| (Q-BioGene™) |
| (MP Bio™) |
| (Norgen™) |
| (RiboGreen™) |
| (Ready to Go™ RT-PCR) |
| (Omniscript™ RT Kit) |
| (RNeasy™ columns) |

Or, les mRNA ne représentent que de 1 à 5% de l'ARN extraite du sol (eg. Mettel et al. 2010). De plus cette ARN est très fragile (Bodrossy et al. 2006), transitoire et sujette à dégradation par les RNases (idem op. cit). Enfin, cette *éphémèralité* de mRNA implique que leur dosage nécessite une activité importante des populations bactériennes (Fleming et al. 1998, Tsai et al. 1991, Mendum et al. 1998, Knauth et al. 2005, Bürgmann et al. 2003), voire la pré - incubation des échantillons de sols et/ou de microcosmes enrichis de grandes quantités de sucres ; Bürgmann et al. 2003 proposent 2 g de sucrose pour 100 g de sol, soit l'équivalant de plus de 25 tonnes de sucrose par hectare, ou encore 100-250 tonnes de résidus de culture pailleux.

Le diagnostic (profilage) des populations bactériennes du sol implique généralement un enrichissement en sucres excessif des échantillons lors de pré-incubations sensé mettre en évidence les caractéristiques de diversité métabolique de ces communautés bactériennes. Cet enrichissement excessif est incompatible avec la cinétique de dégradation des résidus de culture pailleux au sol. Le diagnostique de ces mêmes populations par extraction, purification et dosage des ARN, et plus particulièrement des ARN messagères (mRNA) est fastidieux et nécessairement imprécis en raison de la petitesse des échantillons traitables par rapport aux dimensions de la parcelle agronomique, et de la fragilité des mRNA.

### 2. Diversité génomique des diazotrophes en sols arables

Lors de la (ré)introduction de biomasses (azoto)bactériennes en sols arables, avantageusement via la bactérisation des résidus de culture pailleux au sol(eg. Claude et Fillion 2004, Martin et Brown 1938), il est souhaitable de diagnostiquer l'état de la vie bactérienne du sol. Or, en principe et au niveau génomique, l'appareil diazotrophe, dit ici NIF, est très répandu chez les archéa- et eubactéries (Young et al. 1987 (Phylogenetic classification of nitrogen fixing organisms *;* in Biological Nitrogen Fixation (eds. Stacey et al. 1992), Chapman et Hall Inc. pp. 43-86). L'incidence génomique de diazotrophes dans les sols est donc importante, et pourrait confondre un diagnostique n'ayant pas « fait le tri » entre les NIF suffisamment exprimés pour avoir une incidence agronomique sur l'alimentation en azote des cultures ceux qui ne le sont pas (eg. Bürgmann et al. 2003). *A priori,* il faudrait donc doser les ARN messagers (mRNA), mesure directe de la *transcription* du génome nif codant pour NIF.

Or, la diversité génomique des sols outrepasse celle des souches et/ou espèces microbiologiques « cultivables ». *Idem* donc pour ce qui concerne les populations diazotrophes des sols arables. De plus, ces dernières sont ubiquitaires du fait de la quasi universalité du complexe génomique nif chez les bactéries. En effet, initialement nif était nécessairement présent chez toutes les archea et bactéries (*cf*. Young 1987 ; *supra*) ; il fut par la suite perdu chez certaines par pression évolutive vers une certaine spécialisation. Cela dit, toutes les diazotrophes ne sont pas nécessairement aussi productives, certaines ne conservant nif que comme une forme d'assurance survie en cas de manque complet d'azote assimilable, et cela encore qu'en absence d'oxygène (i.e. très faible pO₂). L'expression à hauts régimes et conséquente en termes agro-écologique de l'ensemble de l'appareil diazotrophe est beaucoup plus limitée. En ce sens, Poly et al. 2001 reconnaissent que la structure de l'ensemble du contingent nifH d'un sol n'est pas nécessairement lié à l'expression de nifH et/ou l'activité in situ de la nitrogenase.

### 3. Expression génomique de la (di)nitrogenase (diazotrophie) in situ

Les *Azotobacteraceae* sont vraisemblablement les seules bactéries capables de maintenir en même temps de hauts taux de glycolyse et d'activité de la *nitrogenase,* cette enzyme étant pourtant particulièrement sensible à l'oxygène et ne fonctionne que si la pO₂ est faible, voire nulle (anaérobie ; Limmer et Drake 1998). Pour ce faire, il semble que les *Azotobacteraceae* sont soit capables de protéger leur appareil diazotrophe (NIF) à l'aide d'une certaine conformation particulière, soit en produisant de copieuses quantité d'exo-polysaccharide (EPS) capable de sanctuariser en sorte la nitrogenase. L'autre grande théorie pouvant expliquer ce fonctionnement à haut régime de NIF en présence de pO₂ élevées suppose une surconsommation de l'oxygène par la cellule avant qu'elle n'atteigne NIF. Certaines *Azotobacteraceae* sont en effet extraordinairement consommatrice d'oxygène, possédant des Qo₂ de l'ordre de 2 400 (Lineweaver 1933), les Qo₂ des autres espèces vivantes, y compris les *Bacillus,* les levure et/ou les cellules mammifère ne dépassant pas 50 à 80, ceux des *Bacillus* étant eux de l'ordre de 2 à 3.

Or, les populations azotobactériennes endogènes sont elles - apparemment, peu nombreuses, soit de l'ordre de 10³ à 10⁴ ufc par g de sol (Roper et al. 1995), soit environ 1 millionième du contingent bactérien du sol. Pire, encore une fois les *Azotobacteraceae* les mieux adaptées à la vie dans les sols s'activent dès l'apparition de la concentration d'oses aussi faibles que que 70 à 100 uM, soit l'équivalant de guère plus que quelques kg de tels sucres par hectare ; à comparer avec les quelques 25 tonnes proposées par Bürgmann et al. 2003. Ce surdosage de substrats carbonés à cependant au moins le mérite de permettre le développement rapide d'une abondance des bactéries du sols dans leur ensemble au détriments d'éventuels contaminants aériens, y compris ceux présents au laboratoire d'analyse. Cela dit, encore une fois, cette surabondance de sucres n'est cependant pas pertinente, les bactéries les mieux adaptées à la vie dans les sols arables étant elles au contraire capables surtout de réagir à de très faibles molarités d'oses.

### 4. Les diazotrophes « réactives » en sols arables

Une fois la diversité et l'expression génomique de la *nitrogenase* du sol diagnostiquées, notamment celles des *Azotobacteraceae,* faut-il encore pourvoir isoler des souches bactériennes réactives métaboliquement dès l'apparition les premiers micromoles de sucres. En effet, les populations bactériennes du sol peuvent être catégorisées comme étant soit zymogènes ou autochtones (Langer et al. 2004). Il s'agit de populations plus (copiotrophes) ou moins (oligotrophes), respectivement, réactives à l'apport d'importantes quantités de substrats facilement assimilables tels que des sucres. Or, l'essentielle de la biomasse bactérienne du sol est, pour cause, plutôt oligotrophes. C'est donc ce segment de la population bactérienne du sol qu'il faut sonder et/ou cibler par des approches de biofertilisations.

Cela dit, certaines biomasses bactériennes oligotrophes réagissent à de très faibles quantités de substrats particulier tels que le glucose (De Nobili et al. 2001, Nguyen et al. 2001, Mondini et al. 2006) et/ou certains acides phénoliques (Wu et al. 1987). Ces populations de bactéries réactives - et ici plus particulièrement diazotrophes, sont donc en étant de « veille métabolique », par opposition à une état de dormance ; elles maintiennent une « charge énergétique » (*adenylate energy charge,* AEC ; Wiebe ett Bancroft 1975, Champman et al. 1971, Kinniment et Wimpenny 1992, Rosaker et Kieft 1990, Martens 1985, De Nobili et al. 2001) très élevée et caractéristique de cellules bactériennes en phase exponentielle de croissance toutes en restant physiologiquement et métaboliquement très stables (i.e. en veille). C'est cet état qui leur permet de « bondir » les premières dès l'apparition d'n minimum de substrat énergétique à hauteur de l'équivalant de quelques 70 à 100 uM (micro-molaire) de d'oses et/ou d'osides. L'avantage écologique d'une telle réactivité en milieux oligotrophique est indéniable.

Malgré l'ubiquité des bactéries diazotrophes dans les sols (Poly et al. 2001, Steward et al. 2004), les plus réactives aux très faibles concentrations de substrats carbonés assimilables dès le début de la dégradation des résidus pailleux sont néanmoins minoritaires. La diazotrophie *in situ* en sols arables est donc rapidement supplantée par d'autres processus microbiologiques moins susceptibles aux stresses oxydatifs (eg. nitrification, dénitrification, ammonification, minéralisation de l'azote et du phosphore organique, etc.). La diazotrophie non symbiotique ne contribue normalement que quelque kg-N par années au bilan azoté du sol. Bien que important en terme écologique, en situations agronomiques cette contribution est généralement reconnue comme négligeable (Roper et al. 1995, Harper et Lynch et al. 1984).

### 5. Protocles d'obtention et de dénombrement des diazotrophes du sol

Janssen et al. (2002) ainsi que WO 2003/042351 mentionnent la mise en culture de bactéries à croissance lente (« *slow growing* ») malgré la présence et la forte concurrence des bactéries dites à croissance rapide (« *fast growing* »), ou « copiotrophes ». Bastiaens et *al.* (2002), eux, utilisent une surface solide immergée dans un milieu de culture riche conventionnel. Cependant, et contrairement à FR0115542, la surface solide est une membrane hydrophobique imprégnée d'hydrocarbures ; la membrane n'est pas l'analogue de l'environnement édaphique. Il existe aussi quelques méthodes, à base de membranes carbonées permettant la mise en contacte d'une solution saline extraite du sol, et de bactéries supposément oligotrophes (Ferrai *et al.* 2005, 2006). Bien que ces méthodes, dites « SSMS » (*soil slurry membrance systems*), permettent de mettre en culture des isolats distincts, variés et précédemment dits « incultivable » *in vitro,* rien ne laisse croire que ces isolats sont particulièrement réactives au sens entendu ci-dessus ; les dites SSMS ressemblent étrangement au procédé décrit dans le brevet allemand DE 21 58 827

Aagot *et al.* (2001) ne cherchent qu'à établir le seuil de dilution d'une des composantes d'un milieu de culture conventionnel au-delà du quel les cellules plutôt oligotrophes peuvent croître au dépends des cellules copiotrophes. La caractérisation des divers isolats ainsi obtenus démontre que les souches isolées en milieux de culture dilués sont différentes de celles isolées en milieux de culture non - dilués. Grundmann et al. 2001 proposent plutôt une « micro-fragmentation » et un « micro - échantillonnage » du sol. Cette micro - fragmentation évite de mettre en concurrence des microorganismes qui, à l'échelle microscopique du moins, n'interagiraient peu ou pas directement ; cette ségrégation spatiale peut favoriser la croissance *in vitro* de microorganismes autrement peu ou pas enclins à le faire étant donné la susdite compétition.

Selon EP 1130115 il est possible de puiser directement à même la diversité bactériologique du sol et d'y trouver spécifiquement des *Azospirillum,* et *Herbaspirillum, etc.* plus ou moins bien adaptées à la vie dans les sols. Il existe aussi des brevets concernant des « supports de récupération » (WO 99/02649), des « méthodes de sélection » (EP 0106504 A2) ou encore des « procédés favorisant la croissance des microorganismes des sols » (WO 99/09834) toutes aussi inadaptées.

FR0115542 concerne l'obtention de bactéries par formation d'un biofilm analogue de la zone hydratée adjointe aux complexes argilo-humiques des sols. Ce biofilm est en sorte une « culture mère de bactéries telluriques » (CMBT). Ces bactéries, avantageusement *Azotobacteraceae, Pseudomanceae* et/ou *Rhizobiaceae,* sont plus plus phytogènes par rapport à l'ensemble des souches témoins. Cependant, étant donné que les CMBT n'abritent pas que des souches bactériennes adaptées à la vie dans les sols, ce processus de sélection par comparaison à des souches témoins, bien qu'efficace, est néanmoins fastidieux. En effet, les quelques souches non - adaptées plutôt copiotrophes, présentes initialement dans les aliquotes provenant des CMBT peuvent déplacer les souches plus adaptées lors de cultures liquides en milieux enrichis.

Enfin, et plus à propos, FR 09/05120 décrit un procédé d'obtention *in vitro* de biomasses bactériennes comprenant, (i) la mise en contacte d'un échantillon de sol avec un substrat matriciel, (ii) la maturation de cette zone permettant aux cellules bactériennes qui ont coloniser ce substrat matriciel de migrer vers la phase liquide le surnageant, et (iii) récupération et culture des souches bactériennes. Le *substrat matriciel carboné, semi-solide* est constitué d'une matrice cellulosique non-bactéricide imprégnée d'eau déminéralisée et stérile placé au fond d'une boîte stérile (bac). Un aliquote de sol dilué sera ajouté de façon à obtenir, entre autres, une teneur en oses initialement inférieure à 70 - 100 uM. Ce substrat matriciel carboné a *priori* dépourvu d'oses et d'osides assimilables par les bactéries du sol en libère progressivement lors de sa dégradation, la microflore d'origine tellurique apportée via l'échantillon de sol assurant la dégradation enzymatique et/ou fongique de la matrice cellulosique.

### 6. Échantillonnage des sols pour diagnostic et obtention microbiologiques

DE 36 32 532 (US4874707) décrit une suspension aqueuse de bactéries réductrices du nitrate. En présence d'oxygène, leur croissance est rapide, bien que leur viabilités réduites ; en présence de nitrate et de nitrite en milieux anaérobiques, leurs délais de conservation sont plus appréciables. L'invention permet de réconcilier ces deux conditions en les « *compartimentalisant* » via un tube étanche dont la paroi n'est que légèrement perméable à l'oxygène. Ce tube est placé dans un contenant dépourvu d'oxygène et contant l'amorce d'une solution aqueuse desdites bactéries dénitrifiantes. Un biofilm aérobie ce formera sur ledit tube ; les bactéries dénitrifiantes pourront ainsi y croître et migrer vers la solution anaérobique où leur viabilité sera assurée. Ce brevet propose donc la *compartimentalisation* de l'oxygène au sein d'un même contenant afin d'assure une croissance initiale, mais surtout la survie à moyen et long terme de bactéries dénitrifiantes ; ladite *compartimentalisation* ne concerne que l'oxygène, et non les phases aqueuses et solides. C'est donc la paroi extérieure de ce tube qui opère cette *compartimentalisation.*

US 6 303 363 décrit la fabrication et l'utilisation de milieux de culture prêts à l'utilisation (« ready to use ») au laboratoire pour détecter visuellement la présence de certains microorganismes dans divers types d'échantillons. Certains de ces échantillons sont placés dans un sachet maillé (filet) afin d'éviter qu'ils ne perforent ou menacent l'intégralité du sac plastique contenant les milieux de culture. Ce sachet semi-rigide et extensible contient un substrat, liquide, pouvant recevoir un échantillon. Si l'échantillon est peu soluble ou perforant, il sera placé dans un sachet (filet). C'est donc filet qui permet de « *compartimentaliser* » l'échantillon et son éventuel substrat. Cela dit, la période d'incubation débutera dès l'introduction de l'échantillon dans le sachet. Il s'agit donc surtout d'un outil pour le contrôle de la qualité (lire : l'absence de contaminants bactériens), un peu en ce sens comme le propose WO97/12029 (voir *infra*).

DE 21 58 827 décrit la conservation et l'utilisation de microorganismes, bactériens en particulier, qui consiste à mettre en contacte un milieu de culture et une membrane microporeuse ne permettant pas le passage desdits microorganismes mais seulement les éléments nutritifs dudit milieu de culture. Or, l'un des côtés de cette membrane est hydrophile et est mis en contacte avec le milieu de culture; l'autre côté est hydrophobe et reçoit les microorganismes dont on veut observer la croissance coloniale afin d'en déterminer le nombre. Le côté hydrophobe étant donc aussi microporeux ne permet pas le passage ver le substrat colloïdale qui le sous-tend des cellules microbiennes ainsi produites et/ou de leurs métabolites pouvant altérer le milieu de culture imprégné dans le susdit substrat colloïdal. Ce substrat peut donc être réutilisé pour recevoir de nouvelles membranes avec de nouvelles souches bactériennes ; la variabilité dans la mesure de dénombrement attribuable à la variation dans la constitution des milieux de culture est donc réduite. Il y a donc « *compartimentalisation* » entre le sol et le les bactéries qui peuvent croître spécifiquement sur ce substrat ; une membrane microporeuse opère cette *compartimentalisation.* Il s'agit donc d'un système semblable à un « microarray », eg. Biolog™, où seules les bactéries présélectionnées pourront se développer à titre d'indicateurs de certaines conditions du sol - substrat (i.e. absence d'azote, présence de certains métaux, etc.).

US 5 155 039 décrit la réactivation de microorganismes conservés par fixation réversible sur un support solide. Ce support, avantageusement le couvercle d'un contenant plastifié hermétique, est mis en contacte avec un milieu de culture liquide, avantageusement contenu dans une fiole sur la quelle peut être vissé ce couvercle. Le milieu de culture liquide est séparé des cellules bactériennes initialement fixées sur le couvercle d'une autre fiole. Il s'agit de transférer ce couvercle vers la fiole contenant le milieu de culture liquide, et de réduire ainsi le risque de contamination lors de l'amorçage de culture à partir de microorganismes lyophilisés, ceux-ci étant parfois un peu « lents » à redémarrer.

WO 97/12029 lui décrit l'utilisation sécuritaire de milieux de culture, avantageusement sélectifs, permettant la détection d'organismes spécifiques dans des échantillons environnementaux. Le procédé combine, (i) une source thermique de fable intensité et de source non - électrique activée lors du transport de l'échantillon, et (ii) un désinfectant solidaire et injectable à être utilisé une fois l'incubation de l'échantillon terminé et, le cas échéant, la présence dudit microorganisme observé. Cette invention s'apparente plus à la fabrication de milieux de culture pour fin de détection de contaminants microbiologiques (*cf. supra* US 6 3030 363) qu'à une méthode de conditionnement et de formulation d'inocula solides non - miscibles à l'eau. S'il y a au sein de ce « réchaud » *compartimentalisation,* c'est plutôt au niveau de la séparation à l'aide d'un écran (*heat shield*) de l'échantillon contenu et d'une quelconque source de chaleur (*heat pack*), plutôt qu'au niveau de l'échantillon et de son substrat.

US 5 905 038 décrit le filtrage des micro-organismes à partir d'un échantillon et leur culture sur une membrane placée sur un support absorbant. Ce filtre est tendu et rétractile de sorte que toute expansion de la membrane filtrante mouillée par l'échantillon n'est pas perturbée par des bulles d'air. Ce bullage inopportun est une véritable problème, mais n'est pas partie intégrante du problème technique résolu par la présente invention. Ce brevet insiste cependant sur l'importance de ne pas avoir à transvaser un échantillons environnemental une fois prélevé de manière à éviter toute contamination par la suite ; l'intégrité apparente de l'échantillon est donc recherchée.

US 5 073 344 décrit un diagnostic dans lequel un substrat poreux est placé dans un récipient. Le substrat sert à extraire un antigène dans ou sur une couche supérieure de celui-ci, le reste du volume du substrat servant de réservoir. Les pores de la surface supérieure du substrat sont microscopiques et piégent les microsphères abritant les anticorps. Un antigène cible dans un échantillon d'essai se fixe aux anticorps lorsque l'échantillon d'essai est versée à travers cette couche supérieure microporeuse. Or, tous les pores du substrat, sauf ceux de sa couche supérieure, sont de plus grande taille de manière à définir la contenance de ce réservoir - substrat. Il s'agit ainsi d'éviter, un peu comme pour US 5 905 038, qu'une quelconque membrane microporeuse placée sur un tel substrat se détache nuisant ainsi au flux capillaire à travers ce filtre. Il est donc question d'échantillonnage de populations d'anticorps, et non de bactéries, d'où l'importance d'un contacte intime et continu entre le substrat - réservoir et l'échantillon séparés que par la membrane, ou couche, microporeuse.

US 5 868 933 décrit une cartouche de filtre antimicrobien dont l'élément de base est perforé et enveloppée avec une première membrane microporeuse, qui est à son tour enveloppé avec les deuxième et troisième membranes microporeuses. Ces membranes sont recouvertes d'une enveloppe de fils enchevêtrés et traités avec un agent antimicrobien. La cartouche filtrant est dimensionnée de manière à s'adapter étroitement à un système de filtration liquide sous forme de boîtier. Le passage de fluides (liquides) à travers la cartouche mise en boîte élimine les microorganismes et, surtout, empêche la croissance des microorganismes bactériens ainsi retenus sur le milieu filtrant. A noter que l'empilage des membranes ne comprend que des membranes microporeuse et non une alternance de membranes micro- et macroporeuses tel que proposé ici.

US 4 299 921 décrit la culture aérobies et anaérobies de microorganismes et de tissus. Deux sections montées l'une sur l'autre à titre de couvercle comportant une mousse filtrante munie d'un joint d'étanchéité lubrifié est recouvert d'un côté par une membrane microporeuse et d'autre part par un revêtement non - poreux. Le joint d'étanchéité est monté sur le couvercle ou sur le rebord du contenant et minimise ainsi le risque de contamination lors de l'incubation de la culture ou du matériel biologique. Il s'agit donc ici d'assurer l'échange gazeux avec la culture microbienne et/ou histologique à l'intérieure de la boîte de Pétri sans provoquer de micro - turbulences capables de contaminer et/ou assécher la culture. Il ne s'agit donc pas de préserver l'intégrité d'un échantillon environnemental lors de son traitement, mais bien de conserver une culture microbio- et/ou histologique active le plus longtemps possible. Ainsi, la disposition des composantes, microporeuses ou non, n'est pas amovible et/ou rétractable tel que proposé.

Enfin, FR2871475 décrit le conditionnement de formulations microbiennes solides et non miscibles à l'eau consistant à réunir la formulation à l'état solide et un contenant devant recevoir la phase aqueuse permettant de re-suspendre les micro-organismes. La méthode permet d'intégrer en un seul procédé de fabrication la fermentation (par multiplication cellulaire) et la formulation à l'état solide, ainsi que le conditionnement en flacon (contenant) verseur. Les cellules microbiennes, avantageusement de type bactérien, une fois produites en nombres suffisants peuvent être formulées au sein de leurs substrats carbonés particulaires et solides.

### DIVULGATION DE L'INVENTION

### Problème technique

Le diagnostique de la diversité et du degré d'expression génomique en sols arables - de la *nitrogenase* des *Azotobacteraceae* par exemple, est problématique. D'un part, l'ARN messagère (mRNA) ne représente que 1 à 5 % de l'ARN normalement présente dans le sol, cette dernière étant elle-même relativement difficile à extraire en raison de contaminants, notamment les acides humiques du sol. De plus, le mRNA dérivés de nifH (mRNA : nifH) sont en l'espèce ubiquitaires et provoqueront ensemble un certain « bruit de fond » capable de masquer le mRNA : nifH provenant des espèces diazotrophes les plus réactives et dynamiques en présence de résidus de culture pailleux au sol. En effet, et selon Young 1987 par exemple, c'est presque 50% des génomes bactériens codent pour la nitrogenase sous une forme ou une autre. Enfin, le protocole d'extraction et de dosage de mRNA - et de l'ADN du coup, dont certains font l'objet de « kit » (*nécessaires*) vendu commercialement (voir *supra*) impliquent tous la manipulation et l'extraction de (très) petites quantités de sols de l'ordre du gramme, généralement frais. De tel quantités exclut *de facto* toute forme d'échantillonnage représentatif (exhaustif, systématique) d'une parcelle agronomique. Sans exiger des échantillons « extractibles » de l'ordre du kilogramme, une centaine de g de sol secs serait nettement plus précis et pertinent.

Le problème technique nait de la nécessité de pré - incuber l'échantillon en présence de très faibles molarités d'oses comme calquées sur celles au début de la dégradation *in situ* de résidus pailleux, avant d'en faire l'extraction en vue d'un dosage soit du titre cellulaire (azoto)bactériens, et/ou des molécules indicatrices de leurs (ré)activité. Normalement, cette pré-incubation nécessiterait l'introduction, au risque de contaminer l'échantillon le substrat au moment choisi pour amorcer ladite pré-incubation. Il fut aussi possible de simplement placer le substrat dans un quelconque bac, et d'y mélanger l'échantillon. Outre les risques évidents de contamination du substrat exposé à l'aire ambiante, c'est surtout l'incapacité à contrôler le moment précis au quel début la pré-incubation qui cause problème. La durée de cette dernière serait au pire égale au délai d'acheminement de l'échantillon ; ce délai d'acheminement sera nécessairement différent pour chaque client et/ou échantillon, sans parler des conditions variables de température, d'humidité, etc. Il serait toujours possible d'instaurer une chaine du froid, mais cela impliquerait des coûts incommensurables avec les prix normalement pratiqués dans le secteur.

L'état de la technique ne permets pas d'assurer simplement et à faible coût, (i) la non-contamination d'un échantillon « environnemental » (**eg**. sol) à mettre en contacte avec un substrat carboné, (ii) la traçabilité, y compris de cette non-contamination, de l'échantillon en question, et (iii) un contrôle précis de la durée et des conditions de (pré-)incubation de l'échantillon en question.

Les « kits » d'échantillonnages proposés à ce jour mettent en oeuvre une double *compartimentalisation* échantillon : substrat. Or, dans il eut été plus souhaitable d'opter pour une triple compartimentalisation échantillon : substrat : contenance. Les « kits » d'échantillonnage et/ou de diagnostique proposés à ce jour sont guères adaptées à la mise en culture et/ou l'obtention de telles bactéries réactives au sens entendu. Le protocole décrit dans FR 09/05120 lui n'est pas facilement mise en oeuvre hors laboratoire et/ou en conditions sceptiques, y compris donc in situ à la ferme. De plus, ce protocole impliquent l'utilisation de petites quantités de sols, trop petites pour être véritablement représentative d'une parcelles agronomique.

De plus, la *traçabilité* de l'échantillon, voire des diagnostiques et/ou des préparations biofertilisantes qui leurs sont propres, est bien perçue par l'agriculteur ; il serait avantageux à ce qu'il puisse apprécier par lui même cette traçabilité. Cela dit, de tels protocoles d'obtention et de diagnostique des populations diazotrophes du sol ne pouvant être menés par l'agriculteur lui-même, ceux-ci devront être effectués « à l'interne » au sein de l'entreprise commerciale. Il serait donc avantageux pour celle-ci de ne pas avoir à rompre le sceaux d'étanchéité aseptique tel que transmis par l'agriculteur ayant lui-même placé dans un quelconque récipient (bac) l'échantillon de sol. Cette intégrité tout au long de l'acheminement de l'échantillon vers l'entreprise contribuera grandement à la traçabilité et/ou la transparence de la procédure de diagnostique. Il serait donc aussi très avantageux, une fois l'échantillon prélevé par l'agriculteur, de ne plus avoir à manipuler directement afin d'éviter de le contaminer, et cela même lors de son incubation. Cela permettrait aussi une économie de moyens, notamment en termes de salles blanches et/ou de flux laminaires.

### Solution technique

La solution technique proposée permettra de combiner deux concepts, notamment ici FR 05/05753 et FR 09/05120, mettant en oeuvre la *compartimentalisation* de l'échantillon et de son substrat carboné, voire cellulosique.

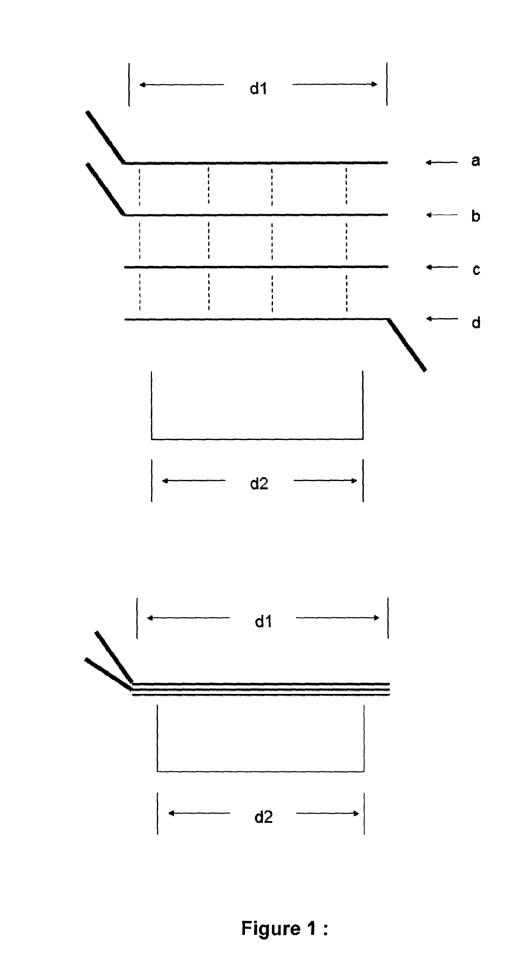

Schématiquement (Figure 1), le feuilleté est représentable par une série de quatre couches ou membranes superposées, les deux couches à l'extérieure du dispositif étant étanches, tandis que les deux membranes à l'intérieure sont soit macro- , soit microporeuses. Les membranes a, b et d sont elles munies de tiges rétractables à la façon d'opercules ; seule la troisième (c) couche ne l'est pas nécessairement. Enfin, ce feuilleté de quatre membranes auto-adhérentes est semi-rigide et de dimension d1 supérieure à celle d'un éventuel bac - avantageusement de forme circulaire, de dimension d2 (Figure 1), et cela de manière à ce que ledit feuilleté puisse être posé sur l'aperture du bac tout en étant fixé par adhésion - par collage par exemple, aux parois extérieures dudit bac une fois la membrane 4 (étanche) enlevée à l'aide dudit tirant rétractible à la manière d'un opercule. Les membranes a et b pouvant par la suite être aussi retirées - séquentiellement, elles aussi à la manière d'un opercule.

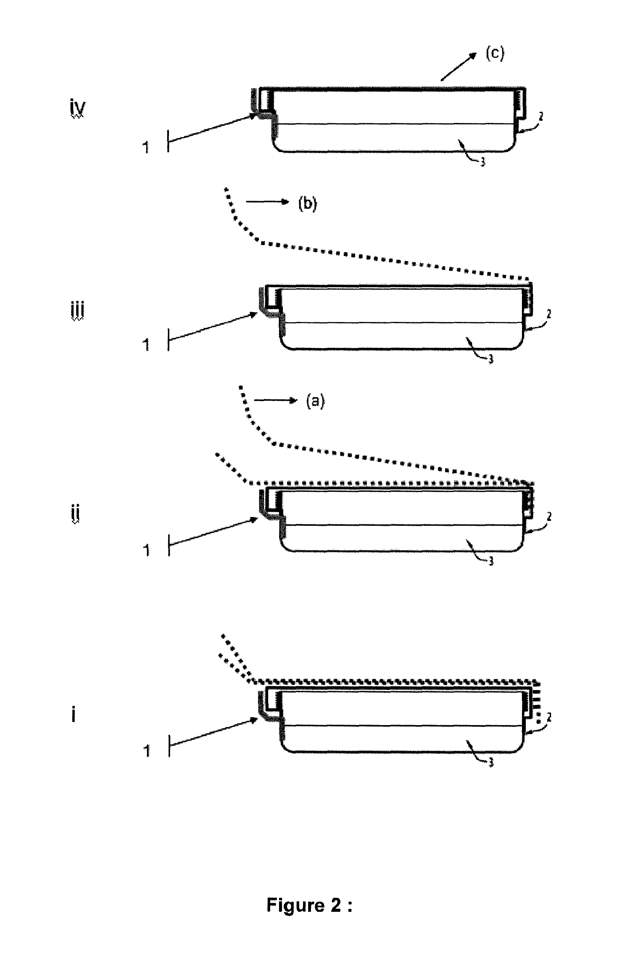

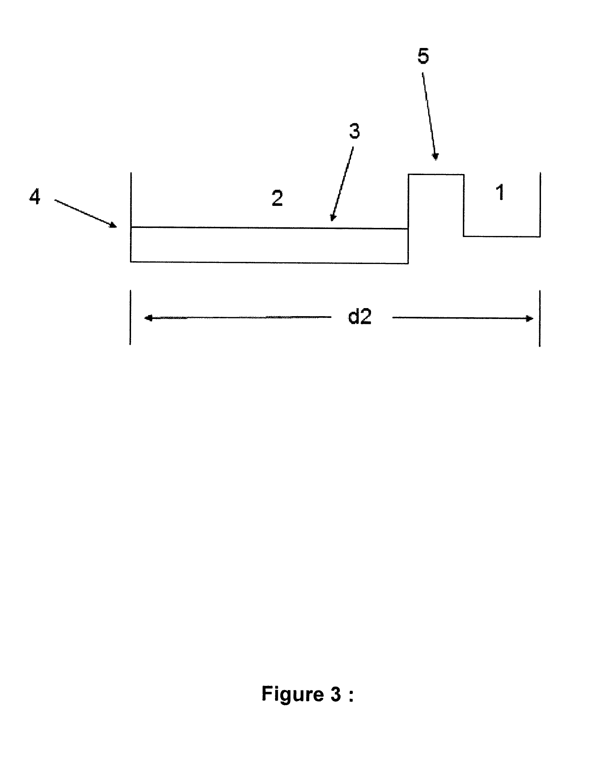

La *compartimentalisation* est assurée par une membrane hydrosoluble placée au dessus d'un substrat cellulosique partiellement enrichie de sucres (ose, osides) de manière à assurer une concentration de sucres exogènes apportés d'au plus 100 uM par unité de volume réactionnel, et cela en accord avec les notions déjà rapportées dans (FR 09/05120) et « OSE » (FR 09/00428). Ce substrat cellulosique micro-granulé (poudre) est préalablement stérilisé fabricant avant d'être confiné par ladite membrane hydrophile (hydrosoluble) au fond du bac (Figures 2 et 3).

Un des modes de réalisation de la solution technique consiste donc à intégrer partiellement les protocoles d'échantillonnage, de diagnostique et de fabrication de préparations biofertilisantes au sein d'un seul « kit » tripartite comprenant ;
(i) un bac pour l'échantillonnage de sols arables contenant une matrice cellulosique recouverte d'une membrane hydrophile,
(ii) un feuilleté multi-plis recouvrant ce bac une fois que l'échantillon de sol y est placé et donc le pli supérieur peut être retiré exposant une membrane macroporeuse filtrante - ce feuillet peut être très avantageusement scellé par l'utilisateur à l'aide d'un code barre autocollant, et
(iii) un contenant étanche pouvant être fixé sur le susdit bac ainsi recouvert et scellé ; c'est dans ce contenant que le diagnostique microbiologique et/ou enzymatique pourra être effectué, et aussi à terme pourra être placé la préparation biofertilisante préconisée qu'en fonction dudit diagnostique.

Cette solution est mise en oeuvre en trois temps par différents intervenants, y compris donc, l'utilisateur final (l'agriculteur) et l'industrie.

Dans un premier temps la parcelle agronomique est échantillonnée, entre 100 et 250 g de sol séché passivement étant placés dans le bac. (Nb. Il est aussi possible d'apporter un échantillon plus petit, de l'ordre d'un ou quelques g ; voir infra). Dans un deuxième temps, l'industriel ayant reçu le bac ainsi scellé peut effectuer un quelconque diagnostique de l'état de la flore diazotrophe du sol, prescrire et fabriquer une préparation biofertilisante « sur-mesure » (voir infra). Sans avoir rompu le scellé, l'industriel peut ainsi fixer ledit bac à un contenant étanche conditionnant cette préparation à renvoyer à l'agriculteur, dans un troisième temps. Il y a donc une forme de consignation « *traçable* » de l'échantillon permettant d'intégrer diagnostique et fabrication de biofertilisants « sur mesure ».

A noter ici que ledit bac - essentiellement similaire à celui décrit dans FR 05/05753 comporte donc deux compartiments. A noter aussi que le dosage du substrat carboné (substrat matriciel cellulosique) est ici différent de celui rapporté dans FR09/05120, le ratio de sol : substrat est plutôt compris entre 25 et 1, plus particulièrement entre 10 et 3, et avantageusement d'environs 5.

Concrètement, il s'agit filtre macro-microporeux constitué d'un feuilleté multi-plis semi-rigide et/ou souple comprenant quatre couches ;
➢ La première (couche a) n'étant pas en contacte avec l'éventuel échantillon est étanche à l'eau et à l'aire ;
➢ La deuxième (couche b) placée immédiatement sous la première est microporeuse du fait d'une maille ou d'une aperture ne dépassant pas avantageusement 0,45 microns (um) et/ou ne permettant pas le passage de cellules bactériennes individuelles ;
➢ La troisième (couche c) placée immédiatement sous la deuxième est macroporeuse du fait d'une maille ou d'une aperture avantageusement d'au moins 70 micron et/ou toute en ne permettant pas le passage de particules de dimensions supérieures 500 um ;
➢ La quatrième (couche d) placée immédiatement sous la troisième est elle soit étanche, soit microporeuse de manière à assurer la stérilité préétablie des deuxième et troisième couches.

Lesdites couches sont constitués de pellicules semi-rigides et/ou suffisamment souples pouvant être fixées l'une à l'autre selon l'ordre établit à la première revendication.
Les couches (a), (b) et (d) sont conçues sous la forme d'opercules rétractibles de manière à permettre de les retirer le moment venu, la couche (c) devenant ainsi à terme orpheline une fois les couches (d), (a) et (b) retirées dans cette ordre respectif. Il est maintenant possible d'apporter - aseptiquement, à l'échantillon de l'eau et/ou une solution nutritive sans avoir à manipuler directement l'échantillon et/ou rompre le lien aseptique qui le protège d'une contamination.

Après y avoir retiré la quatrième couche (d), ledit feuilleté de dimension d1 est placé, à la surface d'un bac - avantageusement circulaire et de dimension d2 inférieure à d1, immédiatement après y avoir placé l'échantillon, le dessous de la troisième couche (c) ainsi exposée étant auto-adhérent de manière à pouvoir se fixer sur les parois dudit bac sur une longueur équivalente à (d1 - d2) / 2. Du coup, les couches (a) et (b) solidaires à ce stade avec la couche (c) sont du coup elles aussi fixées audit bac. La couche (a) est maintenant retirée exposant ainsi la microporosité de la couche (b) surmontant à ce stade la macroporosité de la couche (c) directement en contacte avec l'échantillon dans ledit bac, tandis que la couche (b) est elle par la suite retirée exposant ainsi la macroporosité de la couche (c) toujours en contacte directe avec l'échantillon dans ledit bac. Ledit bac lui contient initialement un substrat cellulosique granulaire enrichit d'oses et/ou d'osides séparé (*compartimentalisé*) de l'éventuel échantillon par une membrane pelliculaire initialement étanche et hydrophile (hydrosoluble). Ce substrat est avantageusement pré-stérilisé au moment de la fabrication du bac. La pellicule hydrophile est aussi assez robuste et/ou épaisse pour résister à l'action perforante de l'échantillon, voire aussi à un minimum d'humidité toujours présente dans ledit échantillon en principe et avantageusement séché passivement avant d'avoir été introduit dans le bac. Il est aussi avantageux de prévoir l'intégration d'un grillage à mailles relativement fines au dessus de la membrane hydrophile de manière à protéger d'autant plus celle-ci de l'action perforante. A noter que la concentration d'ose et/ou d'osides n'équivaut qu'à 70 à 100 uM (micromolaire, p/p) de l'ensemble de l'échantillon et du substrat combinés. Cette concentration (molarité) à respecter est critique puisque, en accord avec FR09/05120 (), c'est elle qui dictera le démarrage de l'activité des bactéries diazotrophes les plus réactives à d'aussi faibles concentration de sucres métabolisables.

Ledit bac ainsi compartimentalisé est par la suite fixé à un récipient de contenance suffisante de manière à permettre ainsi la re-suspension aqueuse des particules de dimension supérieures à au moins 0,45 um (micron) mais inférieures à au plus 500 microns (um) à l'extérieure du bac de manière à pouvoir les récupérer, isoler, obtenir et/ou en déterminer la concentration (dosage). Il est maintenant possible de faire migrer, par suspension aqueuse par exemple, les particules de dimension supérieures à au moins 0,45 um (micron) mais inférieures à au plus 500 microns (um) à l'extérieure du bac de manière à pouvoir les récupérer, isoler, obtenir et/ou en déterminer la concentration (dosage). Plus précisément, le bac auquel adhère toujours les couches b et c dudit feuilleté macro-microporeux filtrant est vissé au récipient sans pour autant rompre l'intégrité desdites couches (b) et (c). Ce vissage, en accord avec le mode opératoire décrit dans FR 05/05753 est possible en raison de la présence d'un pas de vis entourant la paroi extérieure du bac - donc et comme de raison avantageusement circulaire. A noter que le feuilleté semi-rigide, et plus particulièrement la couche macroporeuse (c) restant fixée audit bac n'entrave pas à terme le fonctionnement de ce pas de vis toute en restant intègre.

Le dispositif peut aussi comprendre deux compartiments - deux bacs en sorte (Figure 3), l'un pour la contenance et le dosage bactériologique de l'échantillon, et l'autre - plus petit, pour la détermination des caractéristiques physico-chimique, y compris la teneur en humidité, du sol échantillonné. En effet, le dosage / diagnostique précis de l'échantillon nécessite une détermination de l'humidité du sol afin d'établir exactement le poids sec de l'échantillon. Pour ce faire, et puisque l'échantillon principale doit rester scellé aseptiquement, un deuxième réceptacle (minibac) peut être fixé au bac principale. Ce part sa configuration solidaire audit bac principal plus volumineux, sont remplissage peut ce faire simultanément, quitte à lui apporter moins de sol, soit de l'ordre de 50 à 100 g au lieu des quelques 200 à 300 g à placer dans le bac principal. Le dispositif peut comporter un compartiment axillaire et amovible de plus petite taille pouvant recevoir le moment venu un sous - échantillon frais de sol servant par exemple à la détermination de l'humidité de l'échantillon principale (Figure 3).

### Avantages apportés

Il n'est plus nécessaire de mélanger l'échantillon et le substrat (cellulosique) en salle blanche pour éviter que la pré - incubation (*cf*. FR 09/05120) soit non - contaminée. Le bac + substrat peuvent être fabriqués industriellement et aseptiquement, la mise en oeuvre de la pré-incubation pouvant elle être effectuée en conditions ambiantes non-stériles, d'où une économie de moyens. L'invention se réalise de manière plus transparente, i.e. hors du laboratoire et/ou de l'atelier de l'industriel et en partie par le client-utilisateur lui-même. De plus, le protocole d'obtention décrit dans FR 09/05120 fait donc maintenant partie intégrante d'un kit d'échantillonnage.

L'invention permet d'intégrer le suivi de l'échantillon, de son diagnostique et de la préparation du produit *biodynamisant* de manière à ce que l'utilisateur puisse « reconnaître » par lui-même cette intégration. L'aspect *agro-pédoclimatique* d'un éventuel inoculum diazotrophe (AZB) intégré au susdit produit ne fait qu'accroître cette intégration. L'utilisation pluriannuelle de cette approche procure un suivi de l'état (azoto)bactérien, voire microbiologique de sa parcelle.

### DESCRIPTION DES DESSINS ET FIGURES

**Figure 1** : Représentation schématique du feuilleté multi-plis comportant ici quatre membranes - a, b, c et d, superposées de manière à former qu'un ensemble semi-rigide. Deux de ces membranes sont étanches (a et d), et trois comportent des tirants d'opercules (a, b et d ; voir traits oblique en gras sur la figure). Le feuilleté multi-plis est constitué de quatre (4) couches (pellicules) auto-adhérentes placées les une sur les autres. La première (a), face extérieure par rapport à l'éventuel échantillon, est étanche. La deuxième (b) est elle microporeuse, ne laissant passer que l'aire, l'eau et les molécules et/ou particules inférieures à 0,45 um ; elle assure donc la non-contamination de l'échantillon lors de l'induction (démarrage) de la période d'incubation. La troisième (c) est elle macroporeuse et laissera passer les Azotobacteraceae et autres bactéries su sol mais non les fines particules de sol supérieures à 20, voire 10 um. La quatrième (d) permets de préserver la condition aseptique de l'ensemble des membranes entres a et d, d n'étant retirée par l'utilisateur qu'au moment de poser le feuilleté sur le bac d'échantillonnage (voir infra, Figures 2 et 3). Ce feuilleté multi-plis placé et scellé par l'agriculteur à la surface du bac inférieur peut donc être effeuillé en deux temps ; (i) à la réception de l'échantillon de manière à pouvoir humidifier l'échantillon et amorcer ainsi la période d'incubation, et (ii) à la fin de cette période d'incubation de manière à pouvoir re-suspendre les Azotobacteraceae et/ou autres bactéries diazotrophes pour fin de dosage (dénombrement, dans les faits). A noter que le contenant permettant cette re-suspension est lui vissés sur le bac inférieur prenant ainsi en « sandwich » le dit feuillet. A noter aussi que les liens auto-adhérants (en pointillé sur la figure) ont été ici (schéma du haut) exagérés de manière à mettre en évidence les quatre membranes ; le schéma au bas de la figure est plus condensé et représente une meilleure approximation de l'allure réelle du feuilleté. A noter enfin qu'il est possible de poser - de fixer, le feuilleté au bac en raison de la sur-dimension du feuilleté (d1) par rapport à aux dimensions - avantageusement le diamètre d'un bac circulaire (d2). Les rabats de dimensions moyennes = (d1 - d2) / 2 peuvent maintenant être rabattus et fixés aux parois dudit bac (voir infra Figures 2 et 3). Cette dernière est très avantageusement munie d'un pas de vis (*cf.* Figures 2 et 3), celui-ci ne nuisnat pas en soi à ce stade à l'adhésion des susdits rabats, le feuilleté étant suffisamment fin (flexible et que semi-rigide) à cet effet, tout en pouvant effectivement contenir l'échantillon et son substrat représentant ensembles près de 300 g.
**Figure 2** : Représentation séquentielle de l'apposition initiale (i) des membranes sur le bac une fois la membrane en dessous du feuilleté (d ; Figure 1) enlevée. Dans un premier temps (ii), la membrane par-dessus le feuilleté (a ; Figure 1, en pointillés) est retirée exposant ainsi la membrane microporeuse sous-jacente (b ; Figure 1, en pointillés) adhérant à la membrane macroporeuse en contacte directe avec l'échantillon maintenant en cours de pré-incubation après l'ajout de la solution aqueuse à cet effet. Dans un troisième temps (iii), c'est maintenant cette membrane microporeuse (b ; Figure 1) - et donc à la fin de la période de pré-incubation, qui est enlevée exposant la membrane macroporeuse sous-jacente (c ; Figure 1, en gras et repliée ici par-dessus le pas de vis sur la paroi du bac). Dans un quatrième (iv) temps, la membrane macroporeuse retenant seule l'échantillon pré-incubé séparera ledit échantillon de la contenance d'un récipient pouvant maintenant être - rapidement, fixé audit bac (voir infra Figure 3). A noter sur cette figure le pas de vis par-dessus lequel le feuilleté est rabattu, ainsi que la position du scellé (1). Le bac (2 ; voir aussi Figure 3), ainsi que la position inférieure du substrat (3 ; voir aussi Figure 3) recouvert d'une membrane hydrophile avantageusement renforcée d'un grillage souple afin de contrer l'action perforante de l'échantillon.
**Figure 3** : Configuration alternative du bac de dimension d2 inférieure à celle du feuilleté multi-plis (d1) représenté aux Figures 1 et 2 caractérisé en ce qu'il comporte en plus un compartiment axillaire et amovible (1) de plus petite taille (« mini-bac ») pouvant recevoir le moment venu un sous-échantillon de sol servant à la détermination de l'humidité (teneur en eau) de l'échantillon principale. Ce dernier est toujours placé dans le compartiment principal du bac (2) constitué d'un substrat (3), avantageusement cellulosique, placé au fond du bac et séparé transitoirement de l'échantillon par une membrane hydrophile (4) ; voir la Figure 2 pour les précisions et détails. A noter aussi que la dimension du trait (5) séparant (1) de (2) est suffisant pour que puisse y adhérer une portion de la membrane macroporeuse (c ; Figure 1) suffisamment large pour assurer l'intégrité de l'asepsie de (1) - hors microflore de l'échantillon comme de raison, malgré la rupture de cette membrane au dessus de (2) de manière à pouvoir - dès la réception du bac, retirer, peser et sécher le sous-échantillon.

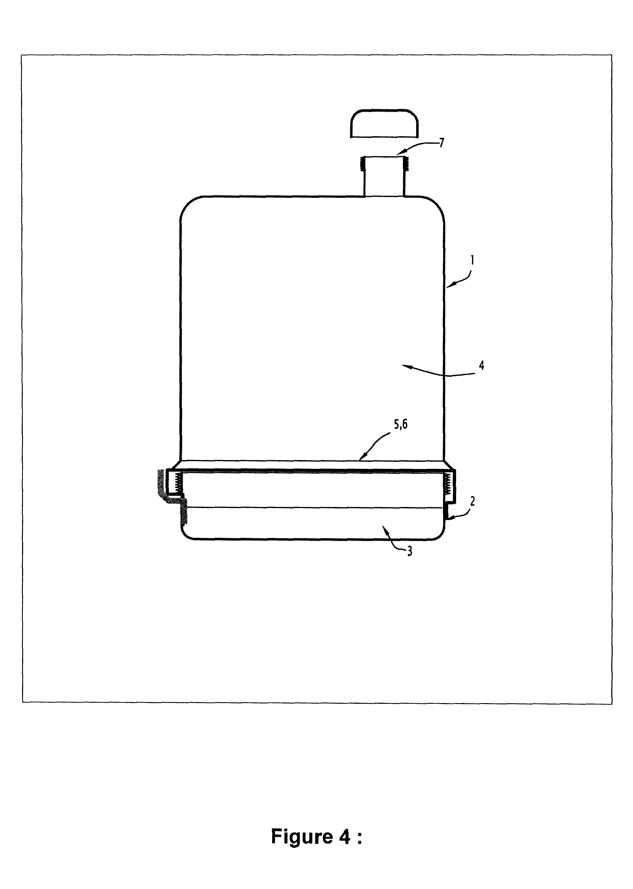

**Figure 4** : Représentation schématique de l'ensemble bac bi-compartimentalisé : récipient de contenance suffisante pour la détermination du nombre et/ou de l'état métabolique des (azoto)bactéries maintenant re-suspendues une fois la pré-incubation terminée. A noter que le récipient (1) d'une certaine contenance (4) est maintenant rempli, via (7), d'une solution de manière à pouvoir extraire par re-suspension de cellules (azoto)bactériennes à travers la membrane macroporeuse. Le volume de la solution - avantageusement aqueuse, est lui établit en fonction du mode opératoire retenu pour le susdit dosage. A noter aussi que cette superposition récipient / bac est en principe analogue à celle proposée dans la demande de brevet FR 05/05753 ; les modalités de réalisation de cette dernière invention sont donc aussi applicable ici, et notamment la fixation dudit récipient via un pas de vis situé sur la paroi du bac. Notamment, il est possible de renforcer l'interface entre la contenance (4) du récipient (1) et la membrane macroporeuse restante (c ; Figure 2) à l'aide d'un grillage (5) suffisamment robuste à cet effet. A noter enfin que ce récipient est avantageusement vissé directement sur le bac, à travers les segments peu épais de la membrane macroporeuse recouvrant maintenant le pas de vis.

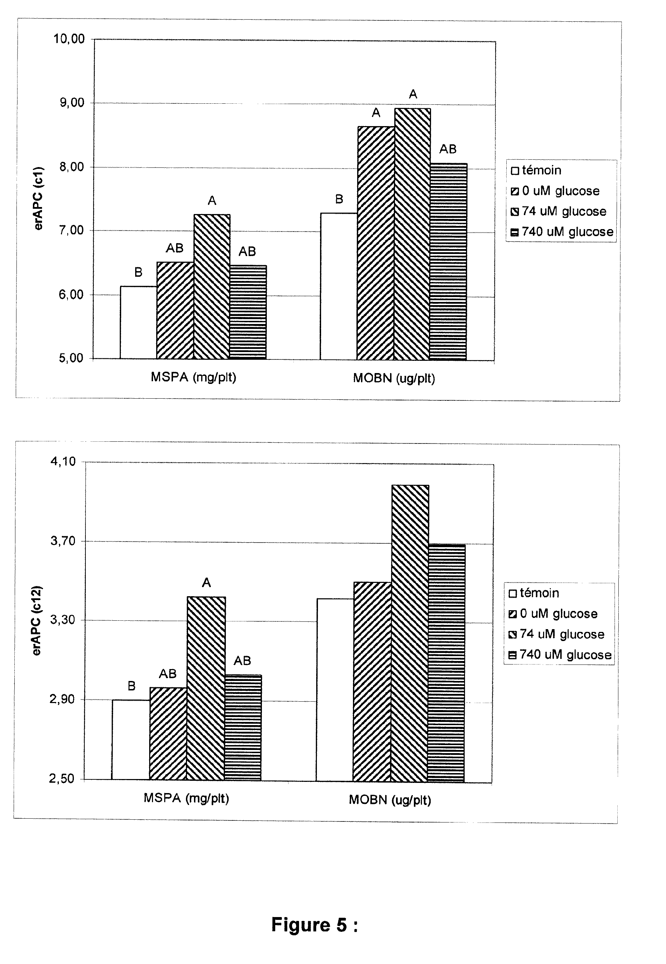

**Figure 5** : *Efficacité agro-pédoclimatique* (erAPC ; sans unités) de populations bactériennes selon la micro-molarité (uM) effective du glucose pendant la période de pré-incubation en termes de MSPA (production de matières sèches des parties aériennes ; mg/plantule) et MOBN (mobilisation de l'azote ; ug-N/plantule) par *Lolium multiflorum* 28 (c1) et 56 (c12) jps (jours post semis), respectivement. Les moyennes associés aux mêmes lettrages ne sont pas appréciablement différentes (Duncan ; α = 5%).

### RÉALISATION DE L'INVENTION

Il s'agit d'aménager au fond d'un bac un volume dans le quel sera déposé un minimum d'ose - avantageusement du glucose, fixé et/ou mélangé à une matrice de cellulose ; la quantité d'ose devra permettre d'atteindre, au plus, une milli-molarité (mM) de 100 lors de l'étape de pré-incubation (EP10366005.6). Ce premier volume d'ose sera contenu par un une membrane hydrophile sur laquelle sera éventuellement dépose l'échantillon de sol provenant d'un échantillonnage systématique de la parcelle agriculteur selon l'état de l'art.

Une fois l'échantillon de sol - avantageusement de 100 à 250 g après séchage passif, placé sur la membrane hydrophile, celui-ci est lui aussi contenu par une membrane cette fois-ci *microporeuse* et temporaire occultée par une pellicule autocollante (*auto-décollante*) fixée aux parois extérieure du bac ; l'utilisateur final (l'agriculteur) pourra à ce moment apposer un premier - de trois, code-barre autocollant de manière à ainsi sceller cette membrane - double épaisseur, au côté du bac. A noter ici que l'agriculteur pourra à terme retrouver sur le conditionnement de la préparation biofertilisante qui lui sera retourné ce scellé à titre de traçabilité.

L'invention est aussi réalisable dans le cadre d'une simple obtention de bactéries par opposition à un dosage de leur activité in situ. Dans ce cas, il faut ajouter que quelques grammes (g) de sol tel que proposé dans EP10366005.6. Dans ce cas, la membrane hydrophile (Figure 2 et 3) abritera une substrat matriciel carboné et semi - solide, et avantageusement constitué d'une matrice cellulosique non - bactéricide, éventuellement imprégnée d'eau déminéralisée et stérile au moment de ladite pré - incubation. Dans ce cas, il faut prévoir une étape de pré - échantillonnage du sol chez l'agriculteur de manière à rendre le prélèvement de ces quelques g de sol pertinent, faute d'être à ce stade véritablement représentatif. De plus, il faut par la suite entrevoir la production par bullage directement dans le contenant de type FR05/05753. Cette production modulaire s'apparente donc à l'idée initiale prônée dans FR05/05753 comprenant une fermentation à l'état solide. Ici, c'est plutôt l'obtention des biomasses biofertilisantes qui s'effectue à l'état solide, leur production en masse état assuré par bullage par la suite.

Une fois le bac ainsi scellé envoyé à l'industriel-prestataire, celui-ci peut retirer la pellicule et fixer la partie supérieure d'un éventuel conditionnement pouvant contenir la préparation biofertilisante, mais avant cela le volume d'eau dans lequel apparaîtra le contingent de diazotrophes les plus réactives à environs 100 uM-C_{glucose} et/ou éventuellement une surconcentration de mRNA : *nif*H*.* Pour obtenir ces populations diazotrophes il faudra simplement hydrater conséquemment l'échantillon de sol ainsi que le substrat sucro - cellulosique sur lequel il repose, laisser pré-incubé, avantageusement en présence d'un minimum de molybdène ; voir EP10366002.3 à ce sujet. A noter ici que cette hydratation de l'échantillon permettra de dissoudre la membrane hydrophile et de mettre ainsi en contacte l'échantillon de sol avec ledit substrat gluco-cellulosiques. La partie supérieure de ce conditionnement hybride est par la suite rapidement remplie d'une quantité d'eau préétablie et agitée ; les titres bactériens (diazotrophes) et/ou les teneurs en mRNA : *nif*H peuvent maintenant être déterminée selon l'état de la technique.

La durée (période) de pré-incubation peut varier selon le but de l'échantillonnage et/ou la précision (résolution) du diagnostique. Cela dit, la présente invention ne concerne que l'échantillonnage applicable à de tels diagnostiques, et non les diagnostiques en soi. Cela dit, la mise en oeuvre d'une précédente invention - EP10366005.6 / FR 09/05120 ayant pour but la production d'un inoculum implique elle une période d'incubation de plusieurs semaines. Un protocole plus dédié au diagnostique qu'à une telle production devra comme de raison impliquer une période d'incubation beaucoup plus courte. A suivre.

Afin de permettre la fixation de la partie supérieure du conditionnement au bac - sans rompre le scellé et/ou l'intégrité de la membrane microporeuse, cette dernière est intégrée à la pellicule double épaisseur surdimensionnée de manière à pouvoir recouvrir la quasi-totalité de la parois du bac, y compris un pas de vis capable de ce fixer sur le pas de vis complémentaire au bas de la partie supérieur ; voir le dispositif proposé dans FR05/05753. A noter que si la pellicule double épaisseur surdimensionnée est suffisamment mince à sa périphérie en contacte avec la parois du bac, celle-ci ne devrais pas entraver le fonctionnement du pas de vis.

### APPLICATIONS BIOINDUSTRIELLES ET AGRONOMIQUES

L'invention est applicable à l'échantillonnage de parcelles et d'îlots agronomiques pour fin de diagnostique de l'état de la vie bactérienne du sol. L'invention permet la traçabilité de l'échantillon, voire de la préparation biofertilisante capable de dynamiser la flore (azoto)bactérienne du sol, moais aussi et surtout d'assurer l'asepsie (lire : non-contamination) lors de la pré-incubation sans avoir recours aux habituelles et coûteuses salles blanches et/ou autres mesure d'exclusion microbiologique.

Pour ce faire, il s'agit d'apposer, dès que l'échantillon (du sol) est placé dans le bac, ledit filtre macro/microporeux après avoir retiré l'opercule que représente la membrane étanche au bas du feuilleté (d ; Figure 1). Ce feuilleté sera recouvert transitoirement d'une pellicule autocollante ; la pellicule et ladite membrane étant apposées solidairement sur le haut du bac une fois l'échantillon de sol (100 à 250 g) placé à l'intérieur. A noter que le module proposé à la Figure 4 est analogue à celui décrit dans FR 05/05753. Ce mode de conditionnement n'est pas nécessairement disponible à l'utilisateur et ne sert qu'à la formulation et au conditionnement d'une préparation inoculante (biofertilisante).

Une fois le bac contenant le ou les échantillons scellé à l'aide d'un code- barre autocollant et unique apposé par l'agriculture et/ou l'utilisateur final, ce bac est renvoyé à l'industriel pour diagnostique et fabrication « sur mesure » de la préparation biofertilisante. Pour ce faire, l'industriel doit retirer la pellicule autocollante recouvrant la membrane supérieure de manière à pouvoir affecter l'échantillon pour l'incubation (Figure 2-ii). En ce sens, l'échantillon ne doit pas occuper tout le volume restant du bas de façon à pouvoir le mélangé (sans rompre le scellé !) au mélange ose/cellulose. Ce dernier est maintenant en contacte directe avec le sol, la membrane hydrophile s'ayant dissoute au contacte de l'eau ajoutée nécessaire à l'incubation.

Une fois l'incubation terminée, le partie supérieure du conditionnement est vissée sur le bac en question (Figure 4), sans rompre le scellé, la pellicule recouvrant le pas de vis inscrit sur le bas ne devant pas enfreindre l'actionnement de ce pas de vis (i.e. la pellicule est somme tout assez mince). Une partie du volume du conditionnement est immédiatement occupé par une solution non-bactéricide (tampon) et agitée de manière à mettre en suspension les bactéries présentes - après incubation, dans le mélange ose/cellulose. Une partie de cette suspension est par la suite échantillonnée pour dosage (mRNA : nifH) et/ou détermination du titre azotobactérien. Selon les résultats de ce dosage - un diagnostique de l'état diazotrophe du sol dans les faits, le volume du conditionnement est complété, soit avec une préparation inoculante, soit avec une préparation biostimulantes (Osâlion™), voire avantageusement les deux.

L'application industrielle et agronomique permets et nécessite l'intégration de concepts déjà brevetés. Cette intégration oblige une certaine *polyvalence redondante a priori* in apparente à l'utilisateur et - surtout, un éventuel contrefacteur. Cette polyvalence redondante opérera à travers le trois stades d'application industrielle et agronomique de l'invention, soit (i) la préparation de l'échantillon à la ferme, (ii) la pré-incubation de l'échantillon, et (iii) la fabrication sur mesure de la préparation biofertilisante ;
(i) L'utilisation d'un bac inférieur - « *vissable* », sur une partie supérieure contenant une préparation biofertilisante fait elle l'objet de FR05/05753. Ce concept sera modifié ici par le placement d'une membrane hydrosoluble séparant transitoirement l'échantillon de sol d'un substrat cellulosique. La présence de ce substrat cellulosique ne contenant qu'un minimum d'ose permettant le « démarrage » des cellules diazotrophes les plus réactives aux oses initialement libérées par les résidusphères fait l'objet de FR 09/05120 ; la membrane hydrophile rendra ce concept plus explicite et défendable. L'agriculteur utilisera un formulaire d'échantillonnage, diagnostique et de préparation « sur mesure » de la formule biofertilisante initialement transmise à l'utilisateur final, i.e. l'agriculteur. Ce formulaire comprend trois autocollants avec le même code - barre unique. L'agriculteur peut signer ce code barre ; le bac ainsi « scellé » sera renvoyé, surmonté du contenant conditionnant la préparation biofertilisante ainsi fabriquée « sur mesure ».
(ii) La pré - incubation pourra elle ce faire en présence d'une quantité spécifique de molybdène (Mo) assimilable de manière à permettre la « suraccumulation » par d'éventuelles diazotrophes les plus réactives (voir supra) de ce Mo ; cette suraccumulation - pondéralement minime par rapport aux usuelles dose-hectare de Mo prescrites en agronomique permet néanmoins de réduire appréciable ces doses effective de Mo. Or cette suraccumulation de Mo est elle en principe couverte par EP10366002. Cela dit, les conditions initiales de cette pré-incubation devront toujours avantageusement satisfaire le non-dépassement du seuil critique aux alentours de 70-100 uM-glucose tel que proposé dans EP10366005.6.
(iii) Une fois le diagnostique établis, soit sur la base de la présence de diazotrophes réactifs à de faibles teneurs en oses, soit via un dosage plus directe de l'expression de leurs ADN - nifH, la préparation biofertilisante sera ajustée - « sur mesure ». Pour ce faire, deux cas de figures ; (a) soit les diazotrophes sont insuffisamment nombreux et il faudra produire les diazotrophes ayant ainsi sur - accumulés du Mo lors de la pré - incubation dans le bas , soit dans le cas contraire il faudra plus simplement préparer une solution biofertilisante sans diazotrophes mais contenant simplement un complément d'oses, de ions calciques, voire de molybdène et de tryptophane. Cette dernière préparation non - inoculante pourra porter la marque de commerce Osâlion™ (marque Polyor SARL).

L'obtention par untel filtrage micro/macroporeux d'un mélange sol : substrat cellulosique dont la micro-molarité (uM) initiale du glucose est établie à environ 100 à déjà fait l'objet d'essais phytogènes non-rapportés à ce jour. En ce sens, un rappel : Dans le cas de la présente invention, cette micro-molarité d'environ 100 uM-glucose, soit en accord avec l'apparent Km pour le glucose des bactéries du sol (Nguyen et al.), est assuré en « coupant » - en sorte, un échantillon de sol de masse appréciable (environ 250 g) avec une masse de substrat cellulosique plus petite d'environ 50 g, et cela sans ajouter de glucose au mélange. De cette façon, la teneur en glucose de l'échantillon - généralement inférieure à 10⁻⁸ g, soit pondéralement environ 10 kg dont environ 500 à 1 000 g en proximité des résidus de culture pailleux enfouis en cours de dégradation (*résidusphère* ; *Nb.* Cette dernière représente en 1 et 10% du poids du sol peu après l'enfouissement desdits résidus pailleux) sera du coup suffisante pour assurer effectivement ce seuil critique au sein du mélange sol : substrat en cours de pré-incubation. Or, dans le cas de la précédente invention (EP10366005.6), cette concentration critique d'oses tel que le glucose est atteinte par ajout de glucose à un échantillon beaucoup plus « coupé » (mélangé) par sont substrat cellulosique (i.e. 0,5 de sol pour 2,5 g de cellulose). D'une manière ou d'une autre, la teneur initiale du (gluc)ose en proximité du substrat cellulosique doit être d'environ 70 à 100 uM pour que les celles (azoto)bactériennes els plus réactives s'imposent lors de la pré-incubation.

Une pré - incubation à de telles concentrations de (gluc)ose favorisera la réactivité des (azoto)bactéries les mieux adaptées à valoriser un apport ponctuel de résidus de culture pailleux au sol, et cela d'autant plus dans un APC spécifique ; voir en ce sens quelques résultats d'essais phytogènes dans EP10366005.6. Du coup, les cellules azotobactériennes ainsi suspendues en solution aqueuse seront non seulement plus réactives et phytogènes qu'autrement, mais aussi - en principe, particulièrement bien adaptées à leur sol, leur APC, d'origine. Il y a donc ici un lien conceptuel entre la réactivité desdites cellules azotobactériennes (AZB) à de faibles teneurs en glucose (*i.e*. 70-100 uM p/p d'oses et/ou d'osides) et leurs efficacité entant que biofertilisants pouvant dynamiser la flore azotobactérienne du sol cible. Cette plus grande efficacité peut être appréciée en établissant un ratio entre l'effet phytogène (*i.e*. production de matière sèche et/ou mobilisation élémentaire, de l'azote notamment, par des plantules tests) sur (dans) le sols d'orgine et/ou provenant d'un même APC et autrement et sur un sols autre provenant d'un autre APC.

Nous avons réévalué ce degré d'efficacité relative APC - spécifique (erAPC ; Figure 5) à partir de donnée obtenues ultérieurement au dépôt initiale de la demande de brevet concernant le concept (i.e. FR09/05120, le 29 octobre 2009). Le protocole de l'essai phytogène est essentiellement le même que celui rapporté dans FR0905120 (voir aussi EP10366005.6). Nous avons donc trois (3) modalités - A, B et C sensées favoriser plus ou moins le développement des *Azotobacteraceae* les plus réactives au sens entendu ;
Modalités A : cellulose avec 0 uM glucose
Modalités B : cellulose avec 74 uM glucose
Modalités C : substrat cellulosique avec 740 uM glucose

C'est ici avec la modalité B (matrice cellulosique + 74 uM glucose et micro-dose Mo) que la flore azotobactérienne semble être la plus phytogène, voire la adaptée à la vie dans les sols en proximité des résidusphère, *i.e*. capable d'une certaine oligotrophie toute en étant très réactive à des apparitions transitoires de très faibles molarité d'oses. De plus - fait notable, cette adaptation à la vie dans les sols est surtout appréciable si le sol ciblé est similaire - voire identique, au sol d'origine d'où proviennent les susdites fines particules de sol partie intégrante de la susdite fermentation à l'état solide. En effet, pour vérifier cette double hypothèse - (i) la supériorité de la modalité C par rapport aux modalités A, B, et C, et (ii) sa plus grande efficacité relative sur sol GEWN d'où proviennent les susdites fines particules de sol par rapport à un autre type de sol, par exemple ici SE13 (Tableau 1 ; EP10366005.6), nous avons installé l'essai en serre suivant.

Les modalités A, B et C avec Mo et appliquées au sol GEWN (Tableau 1 ; EP10366005.6), ainsi qu'un témoin sans inoculation, ont été re-suspendues dans de l'eau déminéralisée (*i.e*. 15 g de préparation inoculante pour 150 mL d'eau), soit une dilution de 10⁻¹ ; 1 mL de cette suspension servant la suite à inoculer 3,5 g de résidus de culture pailleux (blé). En supposant une dose-hectare d'un kg d'inoculum, ce taux d'inoculation pratiqué ici équivaut à l'inoculation *in situ* de 3,5 x 10⁵ g de résidus de culture pailleux au sol par hectare, soit entre 15 et 25 fois moins qu'en situations agronomiques où les retours de pailles et/ou de chaumes sont de l'ordre de 5 à 8 x 10⁶ g par hectare (*cf*. EP10366005.6, Claude et Fillon 2004).

Les 3,5 g de résidus de culture pailleux ainsi inoculés selon les modalités A, B et C et la préparation témoin non - inoculante sont intégrés par malaxage à 350 g de sol sec et reconstitué par tamisage à 4 mm. Deux (2) types de sols de deux APC différentes - « GEWN » (APC no. 29) et « SE13 » (APC no. 40) (*cf*. Tableau 1 ; EP10366005.6) sont utilisés. Le sol GEWN est le sol d'origine d'où proviennent les susdites fines particules de sol intégrées aux fermentations à l'état solide selon les modalités A, B et C. Une fois intégrés au sol, les mélanges sol / résidus de culture pailleux inoculés sont placés dans des pots de 0,5 L ; 4 répétitions (Figure 3) pour chacune des modalités essayées sur sol GEWN et SE13 sont installées. Une quarantaine de graines de *Lolium multiflorum -* soit environ 0,20 g, sont répandues à la surface de chaque pot, et recouvertes de sable siliceux inerte. Les pots sont irrigués avec une solution nutritive contenant cette fois-ci un minimum d'azote minéral (12 mg-N_{NO3NH4} par kg de sol) de façon à éviter aux plantules des carences en N, mais sans pour autant nuire au démarrage de la diazotrophie des *Azotobacteraceae.* Cette solution contient aussi un complément de Mo destiné à la plante, l'alimentation en Mo des *Azotobacteraceae* étant elle assurée par le Mo apporté pendant la fermentation à l'état solide. Enfin, les MSPA du *Lolium* sont récoltées 28 et 56 jours post-semis, séchées à 55-60 degrés C, pesées et analysées pour leurs teneurs en N, P, K et Zn afin d'en déterminer la mobilisation par plantule. Le dosage élémentaire est effectué par le laboratoire d'analyse de la Sadef (www.sadef.fr).

La préparation biofertilisante - à ce stade constituée d'un inoculum bactérien produit à partir des cellules azotobactériennes obtenues lors de l'évaluation de l'état diazotrophique de l'échantillon - ici d'un sol arable dit GEWN (*cf*. Tableau 1, EP10366005.6), est plus efficace lorsque réintroduite dans son APC d'origine (i.e. sol GEWN) qu'autrement (i.e. sol SE13). Graphiquement, l'efficacité relative APC (erAPC) étant ici le ratio entre les MSPA ou MOBN 28 (c1 ; première coupe) et 56 (c12 ; deuxième coupe) jours post-semis (jps ; Figure 5) lors d'essais phytogènes (*Lolium multiflorum*) avec l'un et l'autre des deux sols (eg. erAPC = MSPA_{GEWN} / MSPA_{SE13}). Nous faisons un parallèle entre erAPC et l'aspect « sur mesure » du diagnostique azotobactérien de la parcelle ; i.e. plus l'erAPC des biomasses azotobactériennes ainsi réintroduites est grande, plus les biomasses azotobactériennes ainsi obtenues sont spécifiques au sol d'où provient l'échantillon original. Le diagnostique que permet ces biomasses est donc pertinent.

### Sigles et définitions

**Kit** : Anglicisme désignant un ensemble d'objets, de composants nécessaires à un montage, à une opération donnée. Ex. Un kit d'analyse comprenant une éprouvette graduée, une pipette, un réactif, une palette de couleurs de mesure.
**Traçabilité** : Procédure de suivi d'un échantillon permettant à l'émetteur de retrouver intacte l'échantillon à l'intérieur de récipient ayant servi à son prélèvement. Ledit récipient (bac) est avantageusement scellé à l'aide d'un code-barre autocollant unique.
**Réactivité** (*inductablité*) : Ce dit des bactéries du sol qui sont capable de réagir métaboliquement à une molarité donnée de substrats carbonés assimilable (eg. oses, osides), avantageusement ici in situ (sols arables) de l'ordre de 70 à 100 uM p/p.
**Feuilleté** (quatre couches) (feuillet) : Disposition superposée de plusieurs couches - ici des membranes étanches (1^{ière} et 4^{ième}) et micro- (2^{ième}) et macroporeuses (3^{ième}).
**Micro-poreux** : Ce dit d'une couche et/ou une membrane dudit feuilleté (feuillet) de maille et/ou d'aperture - selon le cas, d'environs 0,45 um (micron ; micromètre) soit capable de retenir au passage d'une solution aqueuse les plus petites cellules bactériennes.
**Marcro-poreux** : Ce dit d'une couche et/ou membrane dudit feuilleté (feuillet) de maille et/ou d'aperture - selon le cas, d'environ 70 um, soit capable de laisser passer l'essentiel des fines particules du sol, y compris donc les bactéries qui y adhèrent.
**Hydrophile (Hydrosoluble)** : Ce dit ici particulièrement des membranes d'apparence plasmifiée toute en étant rapidement dégradé en en présence d'eau. Ces pellicules sont actuellement très utilisées dans la fabrication de pastilles de lessives pré-conditionnées.
**Contenance** : Volume utile (remplissable) d'un récipient (contenant).
***Compartimentaliser**:* Séparation - transitoire ou permanente, d'un récipient (bac), de manière à éviter le contacte immédiate entre deux matières malgré une certaine proxmité.
**Polyvalence redondante** : Possibilité d'intégrer à un concept ou un invention plusieurs autres concepts/inventions de manière à rendre le produit visée nécessairement plus efficace en dépit de divers aléas climatiques et/ou technique.
**Efficacité agro-pédoclimatique** : Ratio entre la production de matière sèche et/ou sa mobilisation élémentaire, notamment de l'azote et du phosphore, par une plantule teste traitée avec une quelconque biomasse (azoto)bactérienne sur (dans) un sol d'origine d'où provient ladite biomasse et dans (sur) un sol autre provenant d'un autre APC.
**Biomasse azotobactérienne (AZB)** : Population de cellules bactériennes aérobies capables de diazotrophie malgré une pression relative de l'oxygène (pO₂) importante. Traditionnellement, et systématiquement, ces biomasses azotobactériennes sont assimilables aux membres de la famille Azotobacteraceae. Cela dit, la présente définition se veut non-exclusive et comprendra toutes les celles bactériennes de ce type.
**Agro-pédoclimat (APC)** : Ensemble de parcelles agronomiques - pas nécessairement contigües, suffisamment similaires en termes agronomique et « pédoclimatique » (lire : (micro)climate du sol) de manière à imposer aux microflores microbiennes qu'elles abritent des conditions écologiques et évolutives comparables. Une population bactérienne provenant d'une parcelle appartenant à un APC et *ré*introduite dans une autre parcelle - parfois relativement éloignée géographiquement, appartenant au même APC est dites endogènes du fait même de cette simple réintroduction.

### Références, bibliographie et brevets pertinents

Aagot, Nina Ole Nybroe, Preben Nielsen, et Kaare Johnsen. 2001. An Altered Pseudomonas Diversity Is Recovered from Soil by Using Nutrient-Poor Pseudomonas-Selective Soil Extract Media. Appl. Environ. Microbiol. 67 : 5233-5239.
Alden, L., F. Demoling et E. Bââth. 2001. Rapid Method of Determining Factors Limiting Bacterial Growth in Soil. APPL. Environ. Microbiol, p. 1830-1838 Vol. 67, No. 4
Bastiaens, L, D. springael, P. Wattiau, H. Harms, R Dewachter, H. Verachtert et L. Diels. 2000. Isolation of Adherent Polycyclic Aromatic Hydrocarbon (PAH)-Degrading Bacteria Using PAH-Sorbing Carriers. Appl. Environ. Microbiol. 66 : 1834-1843.
Bodrossy, L, N Stralis-Pavese, M Konrad-Koszler, A Weilharter, TG Reichenauer, D Schofer et A Sessitsch. 2006. mRNA-Based Parallel Detection of Active Methanotroph Populations by Use of a Diagnostic Microarray. Appl. Environ. Microbiol. 72 : 1672-1676
Bürgmann, H, F Widmer, WV Sigler et J Zeyer. 2003. mRNA Extraction and Reverse Transcription-PCR Protocol for Detection of nifH Gene Expression by Azotobacter vinelandii in Soil. Appl. Environ. Microbiol., Apr. 2003, p. 1928-1935 Vol. 69, No. 4
Campbell, CD, Stephen J. Chapman, Clare M. Cameron, Mitchell S. Davidson et Jacqueline M. Potts. 2003. A Rapid Microtiter Plate Method To Measure Carbon Dioxide Evolved from Carbon Substrate Amendments so as To Determine the Physiological Profiles of Soil Microbial Communities by Using Whole Soil. Appl. Environ. Microbiol. 69 : 3593-3599
Chapman, AG, L Fall et DE. Atkinson. 1971. Adenylate Energy Charge in Escherichia coli During Growth and Starvation. J. BACTERIOI. Dec. 1971 : 1072-1086
Coody, Peter N., Lee E. Sommers et Darrell W. Nelson. 1986. Kinetics of glucose uptake by soil microorganisms. Soil Biol. Biochem. Vol. 18, no. 3, pp. 283-289
Cayuela, M., T. Sinicco et C. Mondini. 2009. Mineralization dynamics and biochemical properties during initial decomposition of plant and animal residues in soil. Applied soil ecology 41 : 118-127
Chen, Yin, Marc G. Dumont, Aurélie Cébron and J. Colin Murrell. 2007. Identification of active methanotrophs in a landfill cover soil through détection of expression of 16S rRNA and functional genes. Environmental Microbiology (2007) 9(11), 2855-2869
Claude, P-P. et L. Fillion. 2004. Effet de l'apport d'un Inoculum bactérien aux résidus de culture de maïs-grain au Sol sur le rendement et la qualité de blés d'hiver panifiables en France. Agrosolutions 15(1) : 23-29.
Degens, BP. 1999. Catabolic response profiles differ between microorganisms grown in soils. Soil Biol. Biochem. 31 475-477
Degens, BP. 1998. Microbial functional diversity can be influenced by the addition of simple organic substrates to soil. Soil Biol. Biochem. Vol. 30, No. 14, pp. 1981-1988
Degens, BP et JA Harris. 1997. Development of a physiological approach to measuring the catabolic diversity of soil microbial communities. Soil Biol. Biochem. 29 : 1309-1320
De Nobili, M., M. Contin, C. Mondini, P.C. Brookes. 2001. Soil microbial biomass is triggered into activity by trace amounts of substrate. Soil Biol. Biochem. 33 : 1163-1170
Ferrari, B.C., S.J. Binnerup et M. Gillings. 2005. Microcolony cultivation on a soil substrate membrane system selects for previously uncultured soil bacteria. Appl. Environ. Microbiol. 71:8714-8720.
Ferrari, B.C., N. Tuluja, K. Stoner et S. Kjellerberg. 2006. Catalyzed reporter deposition - fluorescence in situ hybridization allows for enrichment-independent detection of microcolony-forming soil bacteria. Appl. Environ. Microbiol. 72 :918-922.
FLEMING, JT, WEN-HSIANG YAO, AND GARY S. SAYLER. 1998. Optimization of Differential Display of Prokaryotic mRNA: Application to Pure Culture and Soil Microcosms. Appl. Environ. Microbiol., 64 : 3698-3706
Griffiths, B.S., K. Ritz, N. Ebblewhite, G. Dobson. 1999. Soil microbial community structure: Effects of substrate loading Rates. Soil Biol. Biochem. 31 : 145-153
Grundmann, G. L. A. Dechesne, F. Bartoli, J. P. Flandrois, J. L. Chasse et R. Kizungu. 2001. Spatial modeling of nitrifier microhabitats in soil. Soil sci. Soc. Am. J. 65:1709-1716
Harper, S.H.T. et J.M. Lynch. 1984. Nitrogen fixation by cellulolytic communities at aerobic-anaerobic interfaces in straw. Applied Microbiol. 57:131-137.
Hill, P.W., J.F. Farrar et D.L. Jones. 2009. Decoupling of microbial glucose uptake and mineralization in soil. Soil Biol. Biochem. 40 : 616-624
HURT, RA, X QIU, L WU, Y ROH, AV. PALUMBO, JM. TIEDJE et J ZHOU. 2001. Simultaneous Recovery of RNA and DNA from Soils and Sédiments Appl. Environ. Microbiol., 67 : 4495-4503
Janssen, P.H., Penelope S. Yates, Bronwyn E. Grinton, Paul M. Taylor, et Michelle Sait. (2002). Improved Culturability of Soil Bacteria and Isolation in Pure Culture of Novel Members of the Divisions Acidobacteria, Actinobacteria, Proteobacteria, and Verrucomicrobia. Appl. Environ. Microbiol 68 : 2391-2396.
Kinniment, SL et JWT Wimpenny. 1992. Measurements of the Distribution of Adenylate Concentrations and Adenylate Energy Charge across Pseudomonas aeruginosa Biofilms. Appl. Environ. Microbiol. May 1992 : 1629-1635
Kolb, S, C Knief, S Stubner et R Conrad. 2003. Quantitative Detection of Methanotrophs in Soil by Novel pmoA-Targeted Real-Time PCR Assays. Appl. Environ. Microbiol. 69 : 2423-2429
Kolb, S, C Knief, PF Dunfield et R Conrad. 2005. Abundance and activity of uncultured methanotrophic bacteria involved in the consumption of atmospheric méthane in two forest soils. Environmental Microbiology 7 : 1150-1161
Knauth, S, Th Hurek, D Brar et B Reinhold-Hurek. 2005. Influence of différent Oryza cultivars on expression of nifH gene pools in roots of rice. Environmental Microbiology 7 : 1725-1733
Langer, U. L Böhme et F. Böhme. 2004. Classification of soil microorganims based on growth propeties : a critical view of some commonly useed terms. J. Plant. Nutr. Soil Sci. 167 : 267-269
Limmer, C et HL Drake. 1998. Effects of carbon, nitrogen, and electron acceptor availability on anaerobic n2-fixation in a beech forest soil. Soil Biol. Biochem. Vol. 30, No. 2, pp. 153±158, 1998
Lineweaver, H. 1933. Characteristics of oxidation by azotobacter. JBC
Martens. R. 1985. Estimation of the adenylate energy charge in unamended and amended agricultural soils. Soil Biol. Biochem. 17 : 765-772
Martin, WP et PE Brown. 1938. Inoculation Experiments with Azotobacter on some lowa Soils Soil Sci. Soc. Am. J. 2:271-278.
Mendum, TA R. Elizabeth Sockett, Penny R. Hirsch. 1998. The détection of Gram-negative bacterial mRNA from soil by RT-PCR FEMS Microbiology Letters 164 (1998) 369^373
Mettel, C, Y Kim, P Malla Shrestha et W Liesack. 2010. Extraction of mRNA from Soil. Appl. Environ. Microbiol., Sept. 2010, p. 5995-6000 Vol. 76, No. 17
Mondini, C., M. L. Cayuela . M. A. Sanchez-Monedero, A. Roig et P. C. Brookes. 2006. Soil microbial biomass activation by trace amounts of readily available substrate. Biol Fertil Soils 42: 542-549
Nguyen, C., A. Guckert. 2001. Short-Term Utilisation Of 14C-[U]Glucose By Soil Microorganisms In Relation To Carbon Availability. Soil Biology & Biochemistry 33 (2001) 53±60
POLY, F, L RANJARD, S NAZARET, F GOURBIÈRE et L JOCTEUR-MONROZIER. 2001. Comparison of nifH Gene Pools in Soils and Soil Microenvironments with Contrasting Properties. Appl. Environ. Microbiol. 67 : 2255-2262
Poly, F., L. Jocteur Monrozier et R. Bally. 2001. Improvement in the RFLP procedure for studying the diversity of nifH genes in communities of nitrogen fixers in soil. Res. Microbiol. 152: 95-103
Ranjard, L, F Poly, J Combrisson, A Richaume et S Nazaret. 1998. A single procedure to recover DNA from the surface or inside aggregates and in various size fractions of soil suitable for PCR-based assays of bacterial communities. European Journal of Soil Biology 34 : 89-97
Roper, MM, RR Gault et NA Smith. 1995. Contribution to the N status of soil by free-living N2 fixing bacteria in a lucerne stand. Soil. Biol. Biochem. 27 ; 467-471.
Roper, MM et JK Ladha. 1995. Biological N2 fixation by heterotrophic and phototrophic bacteria in association with straw. Plant and Soil 174:211-224, 1995.
Rosacker, LL. et TL. Kieft. 1990. Biomass and adenylate energy charge of a grassland soil during drying. Soil Biol. Biochem. Volume 22, Issue 8, 1990, Pages 1121-1127
Rowell, MJ. 1995. Colorimetric method for coz in soils measurement. Soil Biol. Biochem. 27 : 373-375. 1995
SAWADA, Kozue, Shinya FUNAKAWA et Takashi KOSAKI. 2009. Threshold concentrations of glucose to increase the ratio of respiration to assimilation in a Japanese arable soil and a strongly acidic Japanese forest soil. Soil Science and Plant Nutrition 55, 634-642
Schneckenberger, K., D. Demin, K. Stahr et Y. Kuzyakov. 2008. Microbial utilization and mineralization of [14C]glucose added in six orders of concentration to soil. Soil Biol. Biochem. 40: 1981-1988
Smalla, K, U Wachtendorf, H Heuer, W-T Liu et L Forney. 1998. Analysis of BIOLOG GN Substrate Utilization Patterns by Microbial Communities. Appl. Environ. Microbiol., 64: 1220-1225
Steward, GF, Bethany D. Jenkins, Bess B. Ward, and Jonathan P. Zehr. 2004. Development and Testing of a DNA Macroarray To Assess Nitrogenase (nifH) Gene Diversity. Appl. Environ. Microbiol. 70 : 1455-1465
TSAI, YU-LI, MJ. PARK et BH. OLSON. 1991. Rapid Method for Direct Extraction of mRNA from Seeded Soils. Appl. Environ. Microbiol. 57 : 765-768
Wakelin, SA, LM Macdonald, SL Rogers, AL Gregg, TP Bolgerd et JA Baldock. 2008. Habitat selective factors influencing the structural composition and functional capacity of microbial communities in agricultural soils. Soil. Biol. Biochem. 40 : 803-813
WIEBE, WJ et K BANCROFT. 1975. Use of the Adenylate Energy Charge Ratio to Measure Growth State of Natural Microbial Communities. PNAS USA 72 : 2112-2115
WU, FJ, J MORENO et GRAVELA. 1987. Growth of Azotobacter vinelandii on Soil Nutrients. Appl. Environ. Microbiol. 53 : 489-494

## Revendications

1. Filtre macro-microporeux constitué d'un feuilleté multi-plis semi-rigide et/ou souple comprenant quatre couches ;
➢ La première (couche a) n'étant pas en contact avec l'éventuel échantillon est étanche à l'eau et à l'air;
➢ La deuxième (couche b) placée immédiatement sous la première est microporeuse du fait d'une maille ou d'une d'aperture ne dépassant pas avantageusement 0,45 microns (um) et/ou ne permettant pas le passage de cellules bactériennes individuelles;
➢ La troisième (couche c) placée immédiatement sous la deuxième est macroporeuse du fait d'une maille ou d'une aperture avantageusement d'au moins 70 microns et/ou toute en ne permettant pas le passage de particules de dimensions supérieures à 500 um ;
➢ La quatrième (couche d) placée immédiatement sous la troisième est elle soit étanche, soit microporeuse.

2. Filtre macro-microporeux selon la première revendication **caractérisée en ce que** lesdites couches sont constitués de pellicules semi-rigides et/ou suffisamment souples pouvant être fixées l'une à l'autre selon l'ordre établi à la première revendication.

3. Filtre macro-microporeux selon un quelconque des revendications précédentes **caractérisé en ce que** les couches (a), (b) et (d) sont conçues sous la forme d'opercules rétractibles de manière à permettre de les retirer le moment venu, la couche (c) devenant ainsi à terme orpheline une fois les couches (d), (a) et (b) retirées dans cette ordre respectif.

4. Bac d'échantillonnage superposé d'un filtre macro-microporeux selon les revendications 1 à 3 précédentes **caractérisé en ce que** le bac contient initialement un substrat cellulosique granulaire enrichi d'oses et/ou d'osides séparé, *compartimentalisé* en sorte, de l'éventuel échantillon par une membrane pelliculaire initialement étanche et hydrophile (hydrosoluble).

5. Bac d'échantillonnage superposé d'un filtre macro-microporeux selon la revendication précédente **caractérisée en ce que** la concentration d'ose et/ou d'osides n'équivaut qu'à 70 à 100 uM (microMolaire, p/p) de l'ensemble de l'échantillon et du substrat combinés.

6. Bac d'échantillonnage superposé d'un filtre macro-microporeux selon un quelconques des deux revendications précédentes **caractérisé en ce que** le bac ainsi *compartimentalisé* est fixé à un récipient de contenance suffisante de manière à permettre ainsi la re-suspension aqueuse des particules de dimension supérieures à au moins 0,45 um (micron) mais inférieures à au plus 500 microns (um) à l'extérieure du bac de manière à pouvoir les récupérer, isoler, obtenir et/ou en déterminer la concentration (dosage).

7. Bac d'échantillonnage superposé d'un filtre macro-microporeux selon la revendication précédente **caractérisé en ce que** le bac auquel adhère toujours les couches (b) et (c) dudit feuilleté macro-microporeux filtrant est vissé au récipient sans pour autant rompre l'intégrité desdites couches (b) et (c).

8. Bac d'échantillonnage superposé d'un filtre macro-microporeux selon la revendication précédente **caractérisé en ce que** le bac comporte en plus un compartiment axillaire et amovible de plus petite taille pouvant recevoir le moment venu un sous-échantillon frais de sol servant avantageusement à la détermination de l'humidité (teneur en eau) de l'échantillon principale.

9. Utilisation d'un filtre macro-microporeux selon les revendications précédentes **caractérisé en ce que** le feuilleté de dimension d1 et semi-rigide est placé, après y avoir retiré la quatrième couche (d), à la surface d'un bac- avantageusement circulaire et de dimension d2 inférieure à d1, immédiatement après y avoir placé l'échantillon, le dessous de la troisième couche (c) ainsi exposée étant auto-adhérent de manière à pouvoir se fixer sur les parois dudit bac sur une longueur équivalente à (d1 - d2) / 2 de manière à ce que les couches (a) et (b) solidaires à ce stade avec la couche (c) soient elles aussi fixées audit bac), que la couche (a) est maintenant retirée exposant ainsi la microporosité de la couche b surmontant à ce stade la macroporosité de la couche c directement en contacte avec l'échantillon dans ledit bac, et que la couche (b) est elle par la suite retirée exposant ainsi la macroporosité de la couche (c) toujours en contacte directe avec l'échantillon dans ledit bac.
